Europäisches Patentamt

European Patent Office

Office européen des brevets

(19)

(11) Numéro de publication: **0 181 273 B1**

(12) **FASCICULE DE BREVET EUROPEEN**

(45) Date de publication du fascicule du brevet:
12.04.89

(51) Int. Cl.⁴: **C 07 D 301/32, C 07 D 303/04**

(21) Numéro de dépôt: **85420188.6**

(22) Date de dépôt: **22.10.85**

(54) Procédé pour concentrer des solutions aqueuses diluées d'oxyde d'éthylène.

(30) Priorité: 31.10.84 FR 8416827

(43) Date de publication de la demande:
14.05.86 Bulletin 86/20

(45) Mention de la délivrance du brevet:
12.04.89 Bulletin 89/15

(84) Etats contractants désignés:
BE DE FR GB IT NL SE

(56) Documents cités:
FR-A- 1 343 492
FR-A- 2 208 892
US-A- 3 217 466
US-A- 3 964 980

Le dossier contient des informations techniques
présentées postérieurement au dépôt de la demande et
ne figurant pas dans le présent fascicule.

(73) Titulaire: ATOCHEM, 4 & 8, Cours Michelet La
Défense 10, F-92800 Puteaux (FR)

(72) Inventeur: Neel, Henry, 9, rue Exelnans,
F-78000 Versailles (FR)
Inventeur: Delannoy, Francis, 61 rue Ampère,
F-69310 Pierre-Benite (FR)

ACTORUM AG

# Description

La présente invention concerne un procédé pour concentrer des solutions aqueuses diluées d'oxyde d'éthylène résultant, dans l'industrie, de l'absorption dans l'eau de l'oxyde d'éthylène contenu dans le mélange gazeux issu de la zone d'oxydation catalytique en phase vapeur de l'éthylène par l'oxygène.

De telles solutions renferment pratiquement jusqu'à environ 7% en poids d'oxyde d'éthylène, généralement moins de 5% et le plus souvent 2 à 3%

Pour obtenir l'oxyde d'éthylène sous forme concentrée ces solutions sont soumises à l'action de la vapeur d'eau dans une colonne à distiller conventionnelle dans laquelle le contact gaz-liquide est assuré, qui comporte généralement 5 à 15 plateaux théoriques, le plus souvent 7 à 12, est munie ou non d'une section d'enrichissement et fonctionne à une pression absolue moyenne comprise entre 1,2 et 6 bars, le plus souvent entre 1,5 et 4 bars.

La solution aqueuse d'oxyde d'éthylène est introduite dans la partie supérieure de la colonne, de préférence le cas échéant sur le dernier plateau.

L'énergie thermique nécessaire à la désorption de l'oxyde d'éthylène est fournie soit par injection de vapeur d'eau au bas de la colonne, le cas échéant sous le dernier plateau de celle-ci, soit au moyen d'un rebouilleur alimenté par une source chaude quelconque.

La colonne délivre en tête un mélange gazeux essentiellement constitué d'oxyde d'éthylène et d'eau et qui contient le cas échéant des gaz initialement présents à l'état dissous dans la solution à concentrer, comme par exemple le dioxyde de carbone et l'éthylène.

La colonne délivre en pied un courant aqueux pratiquement débarrassé d'oxyde d'éthylène et désigné par eau glycolée dans ce qui suit.

Cette eau glycolée peut avantageusement servir, après refroidissement, de courant d'absorption de l'oxyde d'éthylène dans un procédé de fabrication d'oxyde d'éthylène pour former la solution à concentrer.

La teneur en glycol de l'eau glycolée est maintenue à une valeur n'excédant généralement pas 10% en poids par évacuation d'une partie de ce courant hors du circuit absorption-désorption.

Selon l'art antérieur, la solution à concentrer est chauffée à une température de pratiquement 100 °C ou plus avant d'être introduite dans la colonne de désorption et ce, en pratique, par échange thermique indirect avec l'eau glycolée.

C'est ainsi, par exemple, que le brevet des Etats-Unis d'Amérique n° 3 904 656, la demande de brevet français publiée sous le n° 2 305 436 et le brevet français n° 1 343 492 citent, respectivement, des températures de 100 °C, de 98 °C à 104 °C et de 90 °C à 150 °C.

Adoptées dans l'industrie car tenues jusqu'alors comme celles permettant de concilier au mieux efficacité de désorption et économie du procédé, de telles températures et les conditions de fonctionnement propres de la colonne qui leur sont associées ne permettent cependant pas d'éviter une transformation encore trop importante de l'oxyde d'éthylène en produits dérivés et une trop forte dépense d'énergie thermique dans le système même de désorption comme dans celui de dépollution du courant d'eau glycolée évacué hors du circuit absorption-désorption.

Minimiser la transformation de l'oxyde d'éthylène est d'un intérêt évident reconnu par exemple dans la demande de brevet français n° 2 305 436 déjà citée. Malheureusement le procédé proposé dans celle-ci ne permet pratiquement de limiter l'hydratation de l'oxyde d'éthylène que lorsque la teneur en dioxyde de carbone dans le mélange gazeux résultant de l'oxydation de l'éthylène est elle-même limitée. Si tel n'est pas le cas la transformation de l'oxyde d'éthylène est la même que dans les procédés classiques comme celui décrit dans le brevet belge n° 781 107. Dans l'une ou l'autre des hypothèses envisagées dans la demande de brevet français, le procédé qu'elle décrit ne permet pas une économie d'énergie thermique par rapport aux procédés classiques et peut même se traduire par une augmentation de la dépense en énergie.

Le problème technique que résout l'invention est celui de savoir comment intervenir au niveau même du fonctionnement du système de désorption pour en accroître l'efficacité, minimiser la transformation de l'oxyde d'éthylène et économiser de l'énergie thermique.

Le procédé selon l'invention pour concentrer une solution aqueuse diluée d'oxyde d'éthylène résultant de l'absorption dans l'eau de ce produit contenu dans le mélange gazeux issu de la zone d'oxydation catalytique en phase vapeur de l'éthylène par l'oxygène, par désorption de l'oxyde d'éthylène par la vapeur d'eau dans une colonne fonctionnant à une pression absolue moyenne comprise entre 1,2 et 6 bars, est caractérisé en ce que la solution à concentrer est à une température inférieure à 100 °C et comprise entre 70 °C et 100 °C lorsqu'elle est introduite dans la colonne et l'eau glycolée détendue à une pression absolue comprise entre 0,4 et 1,5 bar pour former un courant gazeux qui est introduit dans la colonne pour être utilisé comme fluide de désorption de l'oxyde d'éthylène.

Le procédé de l'invention permet d'éviter les inconvénients normalement prévus si l'on songe à diminuer la température de la solution à concentrer dans le cadre des procédés connus, en particulier l'augmentation de la quantité de produits dérivés de l'oxyde d'éthylène que provoquerait alors un contact à chaud plus prolongé entre l'eau et ce composé dans la colonne, ou l'accroissement de la dépense en énergie thermique à concéder pour maintenir au même niveau le degré d'épuisement de l'eau glycolée en oxyde d'éthylène.

Les domaines de températures et de pressions figurant dans les caractéristiques de l'invention sont ceux permettant au mieux d'éviter les défauts des procédés antérieurs tant au plan économique qu'au plan de l'efficacité intrinsèque de la désorption.

La solution à concentrer est avantageusement portée à la température désirée par échange thermique indirect avec l'eau glycolée.

Le chauffage de la solution à concentrer est évidemment réalisé dans le temps le plus court pour atteindre la température choisie.

La détente de l'eau glycolée est réalisée dans un appareillage classique à pression contrôlée, en une ou plusieurs étapes, généralement trois au plus, avec desurchauffe le cas échéant des vapeurs produites.

Le courant gazeux résultant de la détente de l'eau glycolée est introduit dans la colonne, de préférence sous le dernier plateau le cas échéant, grâce à la mise en œuvre d'un éjecteur ou d'un compresseur de vapeur. Lorsque la pression absolue à laquelle est détendue l'eau glycolée est inférieure à environ la moitié de la pression absolue dans le bas de la colonne, le choix du compresseur de vapeur s'impose pratiquement.

La colonne de désorption comporte généralement, comme dans les procédés de l'art antérieur, de 5 à 15 plateaux théoriques et le plus souvent de 7 à 12.

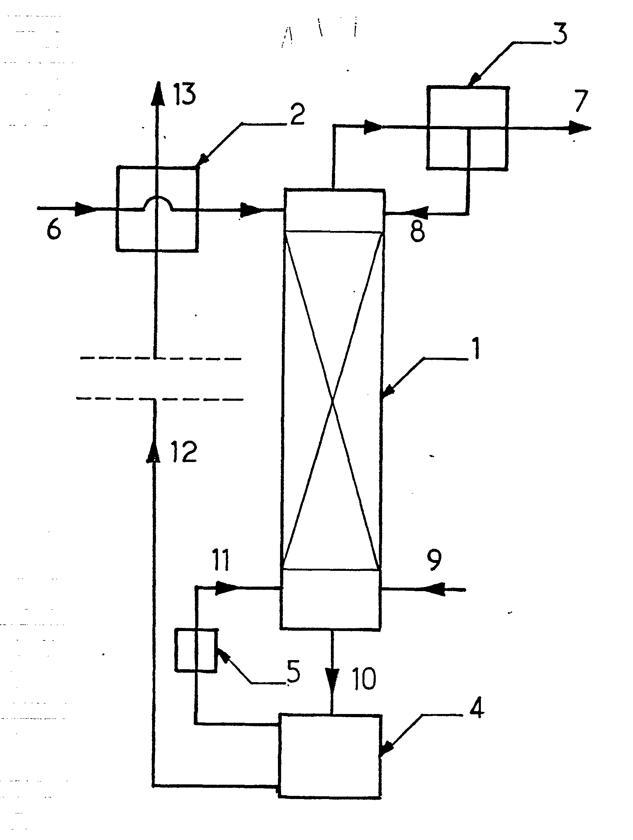

La planche unique dans laquelle, par souci de clarté ne sont pas représentées les parties accessoires telles que pompes, vannes, circuits annexes, etc., montre le schéma d'un dispositif de réalisation de l'invention.

Ce dispositif comprend essentiellement la colonne de désorption 1, des systèmes d'échange thermique indirect 2 et 3 constitués chacun d'un ou plusieurs échangeurs, d'un système de détente 4 et d'un système 5 permettant le retour dans la colonne 1 des vapeurs sortant de 4.

La solution à concentrer circule dans la tuyauterie 6 et est portée dans 2 à la température désirée pour son introduction dans la colonne 1.

Le courant gazeux sortant en tête de colonne par la conduite 7 passe par 3 pour donner un condensat reflué par la conduite 8 et un courant gazeux qui, évacué par 7, pourra être traité de façon connue pour en isoler l'oxyde d'éthylène.

De la vapeur d'eau est injectée par la conduite 9 dans la colonne 1 tandis qu'au bas de celle-ci l'eau glycolée est extraite par la tuyauterie 10 avant d'être détendue en 4.

Le courant gazeux produit en 4 est retourné dans la colonne 1 par la conduite 11 après passage par l'éjecteur ou le compresseur à vapeur 5.

Le courant aqueux sortant de 4 par la conduite 12 est évidemment avantageusement utiliser en 2 pour chauffer la solution à concentrer. La discontinuité de la ligne 12 dans la planche unique explique la possibilité de choix à ce niveau.

Après refroidissement, l'eau glycolée détendue peut bien entendu servir de fluide d'absorption de l'oxyde d'éthylène à partir des gaz le contenant, pour former la solution à concentrer.

Les exemples 1, 2 et 4 illustrent le procédé selon l'invention. Ils sont donnés à titre indicatif mais non limitatif.

Les exemples 3 et 5 permettent de comparer le procédé selon l'invention et un procédé dans lequel l'invention n'est pas comprise. Dans ce dernier cas les conditions de fonctionnement de la colonne de déscription sont celles que le brevet français n° 2 305 436 déjà cité tient pour être les plus répandues dans l'état de la technique qu'il prend en compte.

Sous le terme d'éthylèneglycol sont compris l'éthylèneglycol et les autres composés, en proportion mineure par rapport à lui, qui, comme lui, proviennent de l'oxyde d'éthylène initialement présent dans la solution à concentrer.

La colonne de désorption ayant servi aux essais comporte un nombre de plateaux réels correspondant à 11 plateaux théoriques.

Les numéros figurant dans les exemples se rapportent à la planche unique.

Le courant liquide obtenu après détente de l'eau glycolée est désigné par eau glycolée détendue.

Le courant gazeux fourni par la détente de l'eau glycolée est injecté dans la colonne de désorption à la pression de pied de cette colonne après passage dans un compresseur à vapeur.

Exemple 1

371,584 kg/h d'une solution d'oxyde d'éthylène formée par absorption de ce produit dans l'eau à partir des gaz issus d'un réacteur d'oxydation catalytique en phase vapeur de l'éthylène par l'oxygène, comprenant, en poids:

| | |
|---|---|
| oxyde d'éthylène (OE) | : 2,535% |
| éthylèneglycol (EG) | : 5,31 % |
| eau | : 91,93 % |
| gaz dissous | : 0,22 % |

sont portés de 50 °C à 90 °C en 2 avant d'être introduits sur le dernier plateau supérieur de la colonne 1.

Dans cette dernière, entrent, par 9, 12,703 kg/h de vapeur d'eau à la température de 136 °C et à la pression absolue de 1,5 bar et, par 11, après passage dans le compresseur à vapeur 5, 8,204 kg/h d'un courant gazeux essentiellement constitué de vapeur d'eau résultant de la détente à 1 bar absolu de l'eau glycolée extraite de la colonne de désorption à la température de 111,2 °C et à la pression absolue de 1,5 bar.

L'eau glycolée détendue sort de 4 au débit de 372,731 kg/h à 99,5 °C et à la pression de détente de l'eau glycolée. Elle renferme environ 10 ppm en poids d'oxyde d'éthylène.

Le courant gazeux à la sortie de 3 est à 70 °C, 1,2 bar absolu, a un débit de 11,554 kg/h et la composition pondérale suivante:

| | |
|---|---|
| OE | : 80,15% |
| eau | : 12,66% |
| gaz initialement dissous dans la solution à concentrer | : 7,18% |

Exemple 2

L'exemple 1 est répété, dans le même appareillage, aux différences de fonctionnement près suivantes: la solution à concentrer est portée à 80 °C, 11,245 kg/h de vapeur d'eau sont introduits dans 1 par 9, 385,911 kg/h d'eau glycolée sont extraits de 1 pour être détendus à 0,71 bar absolu et former 14,692 kg/h d'un courant gazeux à la pression de détente et à 90 °C, injecté par 11 dans 1 après compression en 5 et 371,218 kg/h d'un courant d'eau glycolée détendue de mêmes pression et température que le courant gazeux et ne renfermant que moins de 10 ppm en poids d'oxyde d'éthylène.

A la sortie de 3 sont obtenus, dans les conditions de pression et de température de l'exemple 1, 11,639 kg/h d'un courant gazeux contenant essentiellement de l'eau, 80,21% en poids d'oxyde d'éthylène et 7,12% en poids de gaz initialement présents à l'état dissous dans la solution de départ.

Exemple 3 (comparatif)

Les mêmes conditions opératoires que pour les exemples 1 et 2 sont adoptées aux exceptions près précisées ci-après: la solution à concentrer est portée à 100 °C, le débit de vapeur d'eau injecté en 1 par 9 est de 16,948 kg/h, le débit d'eau glycolée est de 377,093 kg/h et l'eau glycolée, qui contient encore 40 ppm en poids environ d'oxyde d'éthylène, n'est pas soumise à détente.

Le courant gazeux sortant de 3, à 70 °C et 1,2 bar absolu comme dans les exemples 1 et 2, a un débit de 11,348 kg/h et contient 80,08% en poids d'oxyde d'éthylène.

La comparaison de l'exemple 3 selon la technique de l'art antérieur, avec les exemples 1 et 2 illustrant l'invention, fait ressortir les avantages de cette dernière: extrême efficacité de la désorption de l'oxyde d'éthylène, transformation minimisée de ce produit en produits dérivés non souhaités, mais habituellement constatés, économie notable d'énergie thermique.

A titre indicatif, il ressort qu'il est possible en procédant selon l'invention de réduire de plus de trois fois la transformation de l'oxyde d'éthylène, d'accroître de deux points environ le rendement de sa désorption et de diminuer simultanément la dépense d'énergie thermique de l'ordre du tiers.

Exemple 4

En procédant comme dans les exemples 1 et 2, mais en portant la solution diluée d'oxyde d'éthylène après 2 à 80 °C, en injectant dans 1, par 9, 9,923 kg/h de vapeur d'eau à 140 °C, 2,9 bars absolus, en soutirant de 1 401,593 kg/h d'eau glycolée à 132,2 °C, 2,9 bars absolus et en les détendant à 0,71 bar absolu pour obtenir 30,8 kg/h de courant gazeux à 90,2 °C et à la pression de détente qui sont retournés à la colonne après compression à la pression de bas de colonne, et 370,788 kg/h d'eau glycolée détendue qui renferme moins de 5 ppm en poids d'oxyde d'éthylène, le courant gazeux extrait par 7 de l'ensemble de désorption à 70 °C, 2,6 bars absolus, a un débit de 10,717 kg/h et contient en poids, à côté de 5,13% d'eau et 7,73% de gaz initialement présents à l'état dissous dans la solution initiale, 87,13% d'oxyde d'éthylène.

Exemple 5 (comparatif)

Dans les conditions qui se différencient ainsi de celles de l'exemple 4: solution à concentrer portée à 115 °C, injection de 20,682 kg/h de vapeur d'eau dans 1 par 9, sortie de 382 kg/h d'eau glycolée renfermant environ 40 ppm en poids d'oxyde d'éthylène et non détente de l'eau glycolée, 10,257 kg/h de vapeurs sont issus de 3, qui contiennent 86,77% en poids d'oxyde d'éthylène à côté de pratiquement les mêmes pourcentages que dans l'exemple 4 d'eau et de gaz initialement dissous dans la solution à concentrer.

La comparaison de l'exemple 4 avec l'exemple 5 montre qu'il est possible, en opérant selon l'invention, par rapport à la technique de l'art antérieur, de réduire de plus de six fois la transformation de l'oxyde d'éthylène, de diminuer de près de la moitié la dépense d'énergie thermique, d'améliorer de 4,5 points environ le rendement de désorption de l'oxyde d'éthylène.

**Revendications**

1. Procédé pour concentrer une solution aqueuse diluée d'oxyde d'éthylène résultant de l'absorption dans l'eau de ce produit contenu dans le mélange gazeux issu de la zone d'oxydation catalytique en phase vapeur de l'éthylène par l'oxygène, par désorption de l'oxyde d'éthylène par la vapeur d'eau dans une colonne à distiller fonctionnant à une pression moyenne comprise entre 1,2 et 6 bars, caractérisé en ce que la solution à concentrer est à une température comprise entre 70 °C et une valeur inférieure à 100 °C lorsqu'elle est introduite dans la colonne et le courant aqueux soutiré au bas de la colonne détendu à une pression absolue comprise entre 0,4 et 1,5 bar pour former un courant gazeux qui est introduit dans la colonne pour être utilisé comme fluide de désorption de l'oxyde d'éthylène.

2. Procédé selon la revendication 1 caractérisé en ce que la solution diluée d'oxyde d'éthylène contient jusqu'à 7% en poids de ce produit.

3. Procédé selon l'une des revendications 1 et 2 caractérisé en ce que la solution diluée d'oxyde d'éthylène contient de 2 à 3% en poids de ce produit.

4. Procédé selon l'une quelconque des revendications 1 à 3 caractérisé en ce que la colonne comporte de 5 à 15 plateaux théoriques.

5. Procédé selon l'une des revendications 1 à 4 caractérisé en ce que le moyen permettant d'introduire dans la colonne le courant gazeux fourni par la détente est un éjecteur ou un compresseur à vapeur.

6. Procédé selon l'une quelconque des revendications 1 à 5, caractérisé en ce que le courant liquide après détente est utilisé pour chauffer la solution diluée d'oxyde d'éthylène par échange thermique indirect.

**Patentansprüche**

1. Verfahren zur Konzentration einer verdünnten wässrigen Lösung von Ethylenoxid, die bei der Adsorption des in einem gsförmigen Gemisch, das aus der Zone der katalytischen Gasphasenoxidation von Ethylen mit Sauerstoff austritt, enthaltenen Ethylenoxids in Wasser resultiert, durch Desorption von Ethylenoxid mit Wasserdampf in einer bei einem mittleren Druck zwischen 1,2 und 6 bar arbeitenden Destillationskolonne, dadurch gekennzeichnet, dass die zu konzentrierende Lösung sich auf einer Temperatur zwischen 70 °C und einem Wert unter 100 °C befindet, wenn sie in die Kolonne eingesetzt wird, und der wässrige Strom unten an der Kolonne auf einen absoluten Druck zwischen 0,4 und 1,5 bar entspannt abgezogen wird, um einen Gasstrom zu erzeugen, der in die Kolonne aus Desorptionsfluidum für das Ethylenoxid eingesetzt wird.

2. Verfahren nach Anspruch 1, dadurch gekennzeichnet, dass die verdünnte Ethylenoxidlösung bis zu 7 Gew.-% dieses Produktes enthält.

3. Verfahren nach Anspruch 1 oder 2, dadurch gekennzeichnet, dass die verdünnte Ethylenoxidlösung 2 bis 3 Gew.-% dieses Produktes enthält.

4. Verfahren nach einem der Ansprüche 1 bis 3, dadurch gekennzeichnet, dass die Kolonne 5 bis 15 theoretische Böden besitzt.

5. Verfahren nach einem der Ansprüche 1 bis 4, dadurch gekennzeichnet, dass das Mittel, das die Einführung des durch die Entspannung gelieferten gasförmigen Stroms in die Kolonne erlaubt, ein Dampfejektor oder ein -kompressor ist.

6. Verfahren nach einem der Ansprüche 1 bis 5, dadurch gekennzeichnet, dass der flüssige Strom nach der Entspannung zur Erwärmung der verdünnten Ethylenoxidlösung durch indirekten Wärmeaustausch verwendet wird.

## Claims

1. A process for concentrating a dilute aqueous solution of ethylene oxide, resulting from the absorption in water of this product contained in the gaseous mixture issuing from the zone of catalytic oxidation of ethylene by oxygen in the vapour phase, by desorption of the ethylene oxide by water vapour in a distillation column operating at a mean pressure between 1.2 and 6 bars inclusive, characterized in that the solution to be concentrated is at a temperature between 70 °C and a value less than 100 °C when it is introduced into the column, and the aqueous flow withdrawn at the base of the column is reduced in pressure to an absolute pressure between 0.4 and 1.5 bars inclusive to form a gaseous current which is introduced into the column to be used as desorbing fluid for the ethylene oxide.

2. A process according to claim 1, characterized in that the dilute solution of ethylene oxide contains up to 7% by weight of this product.

3. A process according to one of claims 1 and 2, characterized in that the dilute solution of ethylene oxide contains from 2 to 3% by weight of this product.

4. A process according to any one of claims 1 to 3, characterized in that the column comprises from 5 to 15 theoretical plates.

5. A process according to one of claims 1 to 4, characterized in that the means enabling the gaseous current provided by the reduction in pressure to be introduced into the column consist of an ejector or a vapour compressor.

6. A process according to any one of claims 1 to 5, characterized in that the liquid current after reduction in pressure is used to heat the dilute solution of ethylene oxide by indirect heat exchange.